# EUROPEAN PATENT SPECIFICATION

(11) **EP 4 751 674 B1**
(45) Date of publication and mention of the grant of the patent: **11.11.2026**
(21) Application number: 24215694.1
(22) Date of filing: 27.11.2024
(51) Int. Cl.: A61B 34/10

(54) **TECHNIQUE FOR AUTOMATIC SPINAL CAGE PLANNING**
TECHNIK ZUR AUTOMATISCHEN PLANUNG EINES WIRBELSÄULENKÄFIGS
TECHNIQUE DE PLANIFICATION AUTOMATIQUE DE CAGE VERTÉBRALE

(43) Date of publication of application: 03.06.2026
(60) Divisional application: 26171152.7 / 4 755 337
(73) Proprietor: Stryker European Operations Limited, Carrigtwohill, Co. Cork T45 HX08 (IE)
(72) Inventor: Weede, Oliver, 79110 Freiburg (DE)
(74) Representative: Schott, Jakob Valentin

(56) References cited:
- US-A1- 2017 252 107
- US-A1- 2018 303 552
- US-A1- 2022 000 625
- US-A1- 20240 374 316

## Description

### Technical Field

The present disclosure generally relates to the planning of spinal surgeries. In particular, a method for automatically planning an implementation of one or more spinal cages, a computer program product, and a planning system are presented.

### Background

Spinal fusion, also called spondylodesis or spondylosyndesis, is a surgery performed by orthopaedic surgeons or neurosurgeons that joins two or more vertebrae. Spinal fusion can be used to treat a variety of conditions affecting any level of the spine, e.g., lumbar, cervical and thoracic. In general, spinal fusion is performed to decompress and stabilize the spine.

There are many types of spinal fusion techniques. Each technique varies depending on the level of the spine and the location of the compressed spinal cord/nerves. After the spine is decompressed, bone graft and/or artificial bone substitute is packed between the vertebrae to help them heal together. In general, fusions are done either on the anterior (stomach), posterior (back), or both sides of the spine.

Most spinal fusion techniques use spine fusion instrumentation, e.g., screws, plates, rods, and/or cages because they have been shown to improve the union rates compared to non-instrumented fusion techniques. Based on the level of the spine and the location of the compressed spinal cord/nerves a variety of different instrumentation, e.g., differently sized and shaped cages may be used.

Which instrumentation is to be used and how to align the spine is commonly manually decided by a surgeon. Since the spine is a highly sensitive area with potentially fatal consequences in case the wrong instrumentation and/or alignment is used, manual planning of which spine fusion instrumentation to use is a very tedious task which can only be performed by an experienced surgeon and which puts a high cognitive load on the surgeon. The manual planning may be based on using a known software for simulating cages like "Surgimap", which allow the surgeon to simulate cages and manually adapt parameters thereof. However, even with such software multiple parameters have to be manually adapted which renders the task to determine an optimized cage extremely complex and tedious or even impossible.

Further, in addition to the manual planning, the suitability of the used instrumentation, e.g., a cage shape, may still have to be evaluated through trial fittings, with fluoroscopic imaging utilized to confirm the placement of the cage. Such trial fittings may result in additional risk for the patient, since suboptimal cages may be used and since the duration of a surgery may be increased. Further, such trial fittings are performed on a patient in a lying position so that a comparison to target alignment derived from a standing x-ray is only possible to a limited extent at best. In other words, a detailed determination of the spinal alignment based on fluoroscopic imaging of a patient in a lying position is impossible. Thus, such trial fittings are only suited to determine whether a cage fits in a disk space between adjacent vertebrae but not whether the target spinal alignment in the standing position will be achieved.

Document US 2017/252107 A1 discloses a system for surgical planning and assessment of spinal deformity correction. The system has a spinal imaging system and a control unit. The spinal imaging system is configured to collect at least one digitized position of one or more vertebral bodies of a subject. The control unit is configured to receive the at least one digitized position, and calculate, based on the at least one digitized position, an optimized posture for the subject. The control unit is configured to receive one or more simulated spinal correction inputs, and based on the inputs and optimized posture, predict an optimal simulated postoperative surgical correction.

Document US 2024/374316 A1 discloses systems and methods for designing and implementing patient-specific surgical procedures and/or medical devices. One method includes receiving a patient data set of a patient. The patient data set is compared to a plurality of reference patient data sets, wherein each of the plurality of reference patient data sets is associated with a corresponding reference patient. A subset of the plurality of reference patient data sets is selected based, at least partly, on similarity to the patient data set and treatment outcome of the corresponding reference patient. Based on the selected subset, at least one surgical procedure or medical device design for treating the patient is generated.

Document US 2022/000625 A1 discloses a method for designing a patient-specific implant. The method includes obtaining image data of a region of interest of the spine of a patient, measuring one or more geometric characteristic of the region of interest from the image data, comparing a measurement obtained for at least one of the one or more geometric characteristics to a mathematical rule associated with the particular geometric characteristic, and generating three-dimensional implant geometry data if the measurement of the at least one of the one or more geometric characteristics conforms with the associated mathematical rule, the implant geometry data configured to guide an additive manufacturing operation.

Document US 2018/303552 A1 discloses systems, methods, and devices for developing patient-specific spinal treatments, operations, and procedures. Some of the systems, methods, and devices can comprise an iterative virtuous cycle. The iterative virtuous cycle can comprise pre-operative, intraoperative, and post-operative techniques or processes. For example, the iterative virtuous cycle can comprise imaging analysis, case simulation, implant production, case support, data collection, machine learning, and/or predictive modelling. One or more techniques or processes of the iterative virtuous cycle can be repeated.

### Summary

There is a need for an improved planning technique addressing at least some of the aforementioned or other problems. In particular, there is need for providing a method for automatically planning an implementation of one or more spinal cages to improve at least one of the size, shape and positioning of the one or more spinal cages to be implanted. Using an automatic planning method may enable evidence based spinal surgery, e.g., to select a spinal cage and alignment that optimize spinal parameters, e.g., that result in spinal parameters that have already been proven to improve the results of a surgery, e.g., at least one of the mechanical stability of the cage(s) and the post operative well-being of a patient and thus result in less revisions. Further, using an automatic planning method may reduce the cognitive load on a surgeon. Summarized, the chances for a successful spinal fusion including post-operative well-being of a patient and mechanical stability of the implanted cage(s) may be increased due to determining an optimized spinal cage implementation, e.g., size, shape and/or positioning.

According to a first aspect, a computer-implemented method for automatically planning an implementation of one or more spinal cages is provided. The method comprises obtaining image data including a 2D medical image comprising representations of a plurality of vertebrae of a patient's body in a standing body posture and determining a 2D prediction image comprising representations of the plurality of vertebrae of the patient's body in the standing body posture, wherein determining the 2D prediction image comprises modifying the 2D medical image such that at least one spinal balance parameter derivable from the 2D prediction image fulfills a predefined criterion. The method further comprises determining, based on the 2D prediction image, an implementation of one or more spinal cages yielding the poses of the vertebrae as represented by the prediction image.

The obtained image data may be or comprise x-ray image data, e.g., a lateral, LAT, x-ray image. The image data may further comprise an anterior-posterior, AP, x-ray image. The obtained image data may be or comprise pre-operative image data allowing the planning to be performed pre-operatively. As a result, for example, the duration of the operation and thus the risk for the patient may be reduced. Additionally or alternatively, intra-operative image data may be obtained allowing for optimization of a planned implementation based on a current spinal shape indicated in the intra-operative image data.

Determining the implementation of the one or more spinal cages comprises transferring the poses of the vertebrae as represented by the 2D prediction image onto vertebrae represented by a 3D patient image, and determining the implementation based on the vertebrae represented by the 3D patient image having the transferred poses. The 3D patient image may be or comprise a computed tomography, CT, scan of a patient.

The method may further comprise determining a registration for registering the 2D medical image with the 3D patient image, wherein the poses of the vertebrae as represented by the 2D prediction image are transferred onto vertebrae represented by the 3D patient image based on the registration of the 2D medical image with the 3D patient image. The registration may be determined based on known registration techniques, e.g., a point to point registration. The registration or landmarks for the registration may be automatically determined using a neural network, e.g., a deep neural network.

The transferred poses of vertebrae of the 3D patient image are further adjusted, in one or more planes differing from an imaging plane of the 2D medical image, before determining the implementation of the one or more spinal cages based on the vertebrae represented by the 3D patient image that have the so-adjusted poses. The further adjustment of the transferred poses may comprise at least one of a cobb angle alignment and an axial alignment of the vertebrae. In some variants, the cobb angle and the axial angle have optimal values (e.g., of zero degrees) and the transferred poses may be determined to be as close as possible to the optimal values.

In some variants, determining the 2D prediction image may comprise simulating a fusion of different vertebra levels, e.g., an L5-L4 fusion and/or an L5-L3 fusion. A prediction image may be determined for each simulated fusion. The fusion of different vertebra levels may comprise a fusion of at least three vertebra levels, e.g., S1-L4, L5-L3, L4-L2, L4-L1 or L3-L1. Additionally or alternatively, the fusion may be simulated based on a predefined maximum number of vertebra levels to be fused.

The method may further comprise (e.g., when at least three vertebra levels are to be fused) outputting a respective optimal fusion result for each number of vertebra levels to be fused, e.g., for three levels, for four levels, for five levels. The method may comprise receiving user-input selecting one of the respective optimal fusion results, and determining the implementation of the one or more spinal cages based on the selected respective optimal fusion result.

Alternatively to the user-input (also referred to as manual input herein) for the selection of the levels to be fused, the fusion levels may be selected automatically. For example, simulating a fusion of different vertebra levels may comprise evaluating all 2-level fusions, followed by the assessment of higher-level fusions. The method may comprise evaluating the results with respect to a predefined threshold indicative of an improvement of the at least one spinal balance parameter resulting from adding another level to the fusion. If the improvement falls below the predefined threshold, the optimal outcome from the fusion with fewer levels may be selected. This systematic approach allows for comprehensive exploration of fusion scenarios and facilitates the identification of the most suitable wedge configuration to optimize spinal alignment and biomechanical stability.

As fusing more levels means a more invasive procedure and restricts mobility of the patient further, determining an appropriate threshold deciding whether it is beneficial to extend the surgery by one additional level may be useful. The method may thus comprise presenting a result of a chosen threshold to a user as a suggestion, said threshold limiting the number of levels to be fused. The suggestion may be adapted based on obtained user input in response to the presentation. As a result, a user, e.g., a surgeon may further specify the number of levels and constrain the automatically determined suggested optimization.

In some variants, simulating the fusion of different vertebra levels may comprise introducing at least one virtual wedge between vertebrae that are simulated to be fused together, for example between vertebrae represented by the 2D medical image or vertebrae represented by a or the 3D patient image. Introducing a wedge may comprise splitting the obtained 2D medical image into at least two parts and moving the at least two parts relative to each other, optionally within 3 degrees of freedom, e.g., two translational degrees of freedom and one rotational degree of freedom. The degrees of freedom may simulate an anterior-posterior translation, a cranial-caudal translation and a lordosis or kyphosis amendment. Each wedge may simulate the shape, size and pose (e.g., at least one of a position and an orientation) of a spinal cage to be implanted. Additionally or alternatively to the relative movement, simulating the fusion of different vertebra levels may comprise moving the 2D prediction image, e.g., rotating the prediction image to simulate a pelvic tilt compensation.

In some variants, the at least one spinal balance parameter may be indicative of at a global alignment and proportion, GAP, score. For example, the at least one spinal balance parameter may be indicative of at least one of a pelvic tilt, PT, a thoracic compensation, a knee flexion, a disc height restauration, an anterior-posterior alignment, a sacral slope, SS, a lumbar lordosis, LL, lumbar lordosis distribution, LDI, a global tilt, GT, a T1 pelvic angle, TPA, a L4-S1 lordosis, a L1 pelvic angle, L1 PA, and a mismatch between a T4 and an L1 pelvic angle, T4PA-L1PA. The at least one spinal balance parameter may be derived from the 2D prediction image. Further spinal balance parameters may include at least one of a sagittal vertical axis, SVA, a T1 spinopelvic inclination, T1 SPI, and a T9 spinopelvic inclination, T9 SPI. Deriving the at least one balance parameter may comprise measuring a value of one of the aforementioned, e.g., a pelvic tilt, in the 2D prediction image and comparing the measured value to an optimal value, e.g., by determining a relative conformity or deviation between the measured value and the optimal value. In other words, a derived spinal balance parameter may be indicative of a relation between a measured value and an optimal value and thus indicative of the quality of the measured value.

In some variants, the method may further comprise obtaining one or more patient specific parameters and deriving the at least one spinal balance parameter based at least in part on the one or more patient specific parameters. The one or more patient specific parameters may be or comprise at least one of a parameter derived from the obtained image data, e.g., based on measuring a pelvic incidence indicated by the image data, metadata associated with the image data or independently provided data. The one or more patient specific parameter may comprise at least one of gender, age and body mass index. Based on the patient specific parameters, upper and lower constraints for values associated with the spinal balance parameters mentioned herein may be defined, e.g., to limit the algorithms for determining the implementation to practically realistic values.

In some variants, multiple spinal balance parameters may be derived from the 2D prediction image and the implementation of one or more spinal cages may be determined based on a combination of at least some of the multiple spinal balance parameters. Combining the at least some of the multiple spinal balance parameters may comprise determining a weighted sum of the derived multiple spinal balance parameters. The method may further comprise determining, based on the weighted sum, a deviation, D(x), of the poses of the vertebrae as represented by the 2D prediction image from an optimal value for the poses of the vertebrae. The deviation may be defined as: $D \left(x\right) = 1 - {Σw}_{i} * {p}_{i} \left(x\right) ; i = 1 , … , n ;$ (1) with D as the deviation, x as a measured value, wᵢ as a weight and pᵢ as a spinal balance parameter. According to the definition of equation (1), pᵢ(x) defines the conformity of a measured value to an optimal value. The above definition for the deviation allows for a fast and easy determination of the quality of the position of the vertebrae as represented by the 2D prediction image and also the quality of specific values allowing a straightforward optimization problem, which may be solved automatically. For example, a deviation of zero, e.g., pᵢ = 1 for i = 1 to n (at least when the weight wᵢ is not zero), is indicative for an ideal condition. In some examples, a parameter pᵢ between 1 and 2/3 may be identified as aligned, e.g., as fulfilling the predetermined criterion, a parameter between 2/3 and 1/3 may be identified as moderately misaligned and a parameter between 1/3 and 0 may be identified as severely misaligned, e.g., as not fulfilling the predetermined criterion. Different alignment interpretations, e.g., different predetermined criteria, are contemplated. For example, the interpretation may comprise a finer or coarser differentiation of alignment states and/or the differentiation between the alignment states may be non-symmetric. In some variants the predetermined criterion may only be fulfilled when the deviation according to equation (1) and/or the deviation between a dedicated measured value and an associated optimal value is smaller than 20%, smaller than 10% or smaller than 5%. Other known mathematical methods for evaluating the quality of the position of the vertebrae as represented by the 2D prediction image are contemplated.

In some variants, deriving the multiple spinal balance parameters from the 2D prediction image may comprise determining multiple spinal balance parameters sets and selecting one of the sets for determining the implementation based on at least one optimization criterium.

In some variants, determining the implementation of the one or more spinal cages may comprise at least one of determining a position and orientation of the one or more spinal cages and determining a respective shape for each of the one or more spinal cages based at least in part on the 2D prediction image or the 3D patient image comprising the transferred poses. In other words, the position, orientation and shape of each of the one or more spinal cages may be determined so that a spine of the patient, after an implementation of the one or more spinal cages, corresponds to the spine indicated in the 2D prediction image or the 3D patient image comprising the transferred poses, e.g., based on the virtual wedges introduced in the images that result in an optimized deviation as explained above.

In some variants, the method may further comprise selecting, based on the determined respective shapes, a spinal cage from a family of predetermined spinal cages matching the determined respective shape the best. Additionally or alternatively, the method may comprise outputting instructions for manufacturing a spinal cage that matches one of the determined respective shapes.

In some variants, determining the implementation of the one or more spinal cages may comprise determining, based at least in part on the 2D prediction image or the 3D patient image comprising the transferred poses, a region within a space between two adjusted vertebrae to place one of the one or more spinal cages in, and determining a position for said spinal cage within the determined region based at least in part on the 2D prediction image. This may be done for each of the one or more spinal cages, for example such that each region is virtually filled by a respective spinal cage.

In some variants, the method may further comprise triggering display of a visualization indicating the determined spinal cage implementation together with the selected spinal cage. The visualization of the selected spinal cage may comprise a visualization of one or more dimensions of the cage, like length, height, width and lordotic angle, and/or a serial number of the cage. The visualization may further comprise indicating the optimal fusion levels of the plurality of vertebrae, e.g., Sᵢ-Lⱼ or Lᵢ-Lⱼ.

In some variants, the visualization may comprise at least one of a visualization in the 2D prediction image, a visualization in the 3D patient image comprising the transferred poses, and a visualization of the selected cage attached to a cage insertion instrument. The visualization described herein may be triggered to be displayed on one or more displays.

In some variants, the method may further comprise receiving user-input adjusting at least one of the visualized spinal cage implementation and the selected spinal cage, and re-calculating the spinal cage implementation and the spinal cage shape based on the user-input. The user-input may indicate specific levels of vertebrae to be fused or a specific number of vertebra levels to be fused. The received user input may adjust specific parameters pᵢ and/or the weights wᵢ thereof.

According to a second aspect, a computer program product is provided, comprising instructions that, when executed on at least one processor, cause the at least one processor to carry out the method described herein. In particular, the instructions, when executed on the at least one processor, cause the at least one processor to carry out any of the variants of the method according to the first aspect.

According to a third aspect a planning system is provided comprising at least one processor configured to carry out the method described herein. In particular, the at least one processor is configured to carry out any of the variants of the method according to the first aspect.

In some variants, the planning system may further comprise at least one of the following entities: a medical imaging device configured to generate at last one of the 2D medical image and the 3D patient image, a user input device configured to receive the user-input, a display device configured to display the visualization.

### Brief Description of the Drawings

Further details, advantages and aspects of the present disclosure will become apparent from the following embodiments taken in conjunction with the drawings, wherein:
- Fig. 1: shows a planning system for automatically planning an implementation of one or more spinal cages;
- Fig. 2: shows a flow diagram of a method for automatically planning an implementation of one or more spinal cages;
- Fig. 3A: shows a schematic example of 2D image data indicating a spine of a patient before the implementation planning;
- Fig. 3B: shows a schematic example of a 2D prediction image;
- Fig. 4A: shows an image of a suggested spine cage implementation with an indication of different spinal balance parameters as well as a user interface for adjusting the suggested spinal cage implementation; and
- Fig. 4B: shows a 3D prediction image and a 2D prediction image indicating a planned spinal cage implementation and spinal balance parameters.

### Detailed Description

In the following description of exemplary embodiments, the same reference numerals are used to denote the same or similar components.

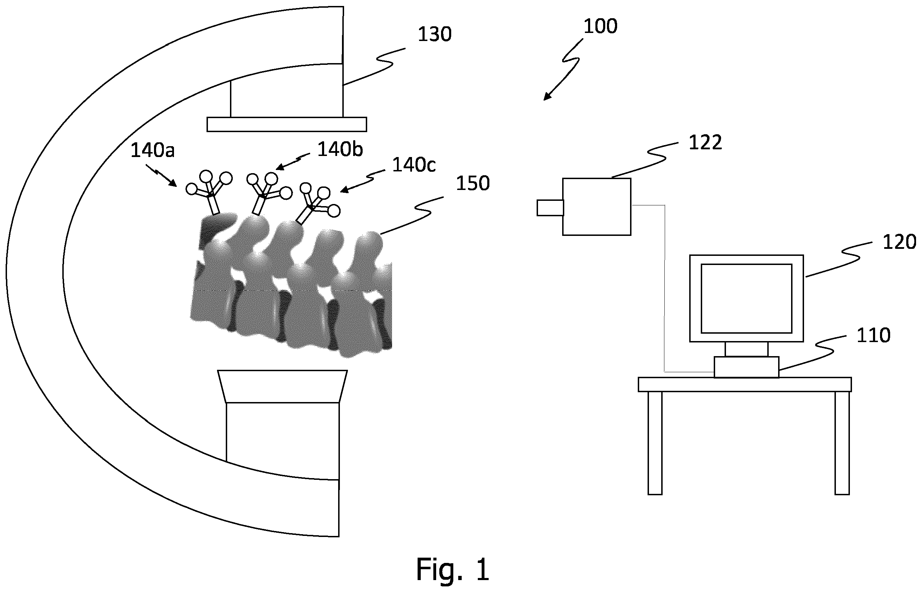

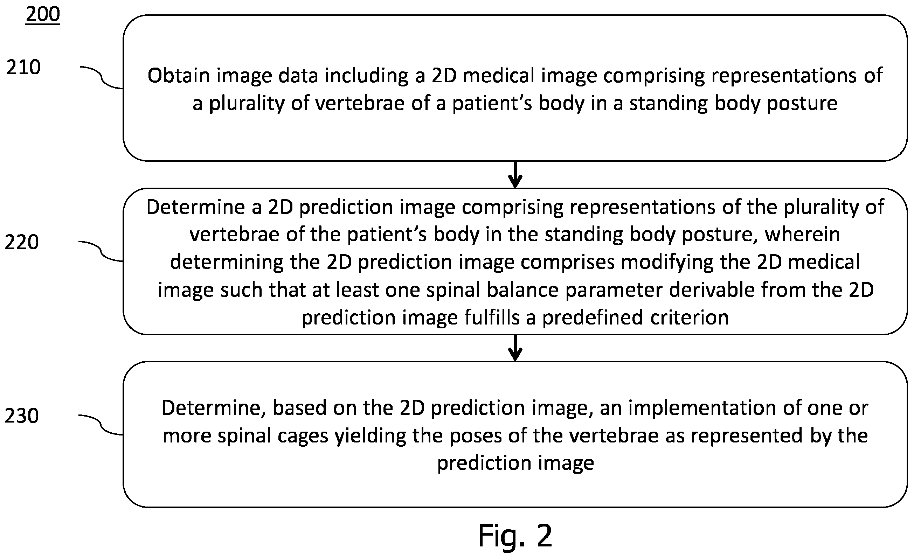

Fig. 1 shows a planning system 100 for automatically planning implementation of one or more spinal cages. The planning system 100 comprises a computing device 110 having a processor configured to carry out the method as explained in detail with reference to Figs. 2 to 4B below. The computing device 110 may be any known computing device 110 comprising one or more processors, e.g., a personal computer, or a server.

The system 100 further comprises a display device 120 for displaying data associated with the planning. In some variants, the display device 120 may be or comprise a touch display configured for receiving user input. Additionally, or alternatively, the planning system 100 may comprise a dedicated user input device (not shown in Fig. 1), e.g., a computer mouse and/or keyboard. Moreover, the system 100 comprises a tracking camera 122 configured for generating tracking data that may be used for intra-operative registration and/or navigation purposes. For example, a planned spinal cage may be visualized within medical image data during implantation of the planned spinal cage based on a registration determined based on the tracking data. The visualization of the planned spinal cage may be used for at least one of navigation purposes, determining whether the planned alignment is achieved in practice, and determining an adapted position for achieving the planned alignment.

Still further, the system 100 comprises a medical imaging device 130 for generating 2D and/or 3D medical image data, e.g., x-ray images or CT scans. The shown medical imaging device 130 comprises a common C-arm. Other known imaging devices, e.g., devices comprising an o-arm, are contemplated.

Moreover, the system 100 comprises multiple optical trackers 140a, 140b 140c trackable via the tracking camera 122. Each of the trackers 140a, 140b 140c is attached to a different vertebra allowing the tracking camera 122 to generate the tracking data for the tracking/navigation/further determination purposes explained above.

Fig. 2 shows a flow diagram of a method 200 for automatically planning an implementation of one or more spinal cages. The method 200 comprises a first step 210 of obtaining image data including a 2D medical image comprising representations of a plurality of vertebrae of a patient's body in a standing body posture.

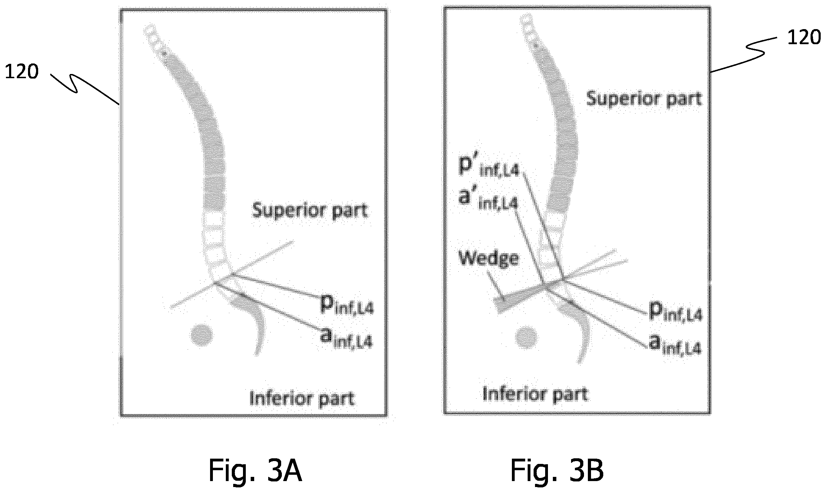

Fig. 3A shows a schematic example for a 2D medical image that can be displayed on the display 120 of the system 100. In the shown example, the spine is separated into a superior part and an inferior part as indicated by the line drawn at the bottom of the vertebra L4, e.g., between the vertebrae L4 and L5, wherein a position p_{inf,L4} of a lower posterior edge and a position a_{inf,L4} of a lower anterior edge of the vertebra L4 are indicated for illustrative purposes. Such a LAT 2D medical X-ray image may be obtained in step 210, the division into the superior and inferior part may be automatically determined based on a segmentation of this image.

Returning to Fig. 2, the method 200 comprises a second step 220 of determining a 2D prediction image comprising representations of the plurality of vertebrae of the patient's body in the standing body posture. Determining the 2D prediction image comprises modifying the 2D medical image obtained in step 210 such that at least one spinal balance parameter derivable from the 2D prediction image fulfills a predefined criterion.

A schematic example of a 2D prediction image displayed on the display 120 of the system 100 is shown in Fig. 3B. In this example, the superior part of the spine as defined in Fig. 3A has been rotated relative to the inferior part as defined in Fig. 3A. In detail, the superior part has been rotated around the indicated posterior point p_{inf,L4}. As a result, a gap in the form of a wedge has been introduced between the vertebrae L4 and L5 as indicated in Fig. 3B. The form of the wedge may be determined based on a spatial relation between the positions p_{inf,L4} and a_{inf,L4} of the indicated edges prior to the rotation and positions p'_{inf,L4} and a'_{inf,L4} after the rotation. While in the shown example only the single rotation as explained above has been performed for clarity purposes, the modification of the 2D medical image may comprise a rotation and one or more translations between two adjacent vertebrae, e.g., between a vertebra pair. Further, respective rotations and translations may be performed between respective vertebra pairs, e.g., between two or three or more respective vertebra pairs, so that not only a superior and inferior part of the spine are defined but multiple parts. The respective rotations and translations may be different for each respective vertebrae pair resulting in respective wedges between the vertebrae pairs.

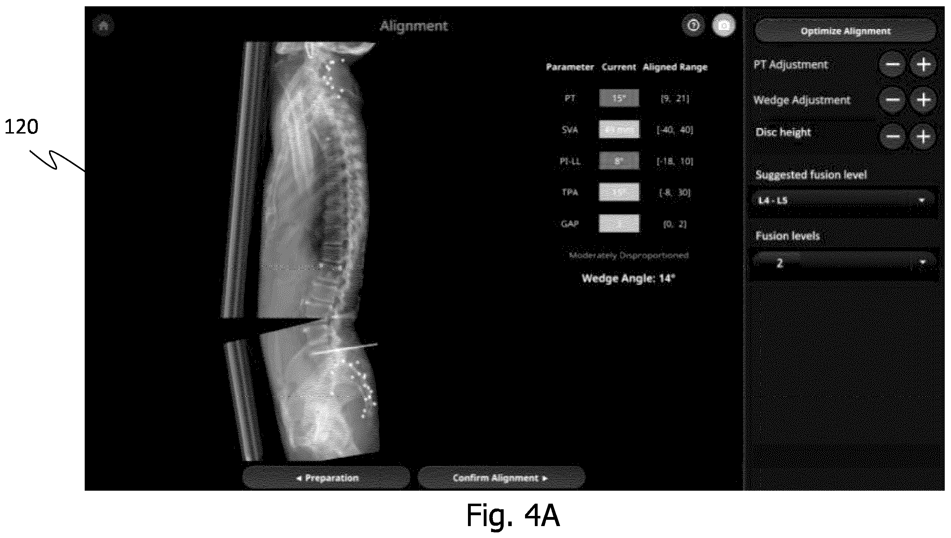

Based on the 2D prediction image, balance parameters pᵢ may be derived as described herein, e.g., with reference to equation (1). Fig. 4A shows a set of derived balance parameters displayed next to a 2D prediction image. In detail, the names of the parameters of the parameter set are shown next to measured values associated with respective parameters of the parameter set. Each measured value is displayed within a window having a colored background indicating whether the parameter fulfills the predefined criterion, e.g., based on a comparison between the shown measured value and a predefined optimal value associated with the respective balance parameter. For example, a green background indicates that the predefined criterion is fulfilled while an orange and/or a red background indicate that the predefined criterion is not-fulfilled. The relative difference between the shown measured value and an optimal value may also be indicated by the colored background, e.g., with green indicating a small relative difference, e.g., smaller than 33 % orange indicating a medium relative difference, e.g., between 33 % and 66 % and red indicating a great difference, e.g., greater 66 %. The indications may comprise more or less or different colors and may be based on different ranges than the ones of the previous example. The optimal values may be defined based on the GAP score.

Further an upper and a lower threshold for the measured values are shown in Fig. 4A. These thresholds are predefined constraints to exclude theoretically possible but practicably non-possible values from the automated determination steps to optimize the implementation planning.

Further, Fig. 4A shows a section of the display 120 configured as a touch display for receiving user input. The user input may define an adjustment of the prediction image. The shown adjustment options comprise a PT adjustment, a wedge adjustment (e.g. an adjustment of the opening angle of the wedge) and a disc height adjustment (e.g., an adjustment of the height and position of the wedge). The aforementioned adjustments may result in an adjusted rotation and/or adjusted translations between two neighboring vertebrae. Further the user input may define the number of spine levels that are to be fused and, still further, may explicitly define which spine levels are to be fused. After receiving user input, a re-planning based on the adjusted parameters may be triggered.

Returning again to Fig. 2, the method 200 further comprises a third step 230 of determining, based on the 2D prediction image, an implementation, e.g., the form position and orientation, of one or more spinal cages yielding the poses of the vertebrae as represented by the prediction image. For example, a region between two vertebrae in which a spinal cage may be optimally located may be determined based on a wedge as shown in Fig. 3B. Determining the region may comprise limiting the wedge based on predefined boundaries, e.g., based on a planned surgical approach and/or to prevent a protrusion of a spinal cage between the vertebrae, when the spinal cage is in the optimal position. Based on the determined region, a form, position and orientation of a spinal cage may be determined. The implementation of one or more spinal cages may further be determined based on additional information determinable from a 3D prediction image, e.g., a length of a disc space between two adjacent vertebrae. For example, the implementation for a lateral spinal cage may be determined based on a length of a disc space along a transversal axis thereof. The form of the spinal cage may comprise a lordotic angle, a cage height, a cage length and a cage width.

While the examples shown in Figs. 3B and 4A above are directed at a 2D prediction image, the poses of the vertebrae in the 2D prediction image may be transferred to 3D image data and the determinations explained with reference to the 2D image data could analogously be performed based on the 3D image data having the transferred poses.

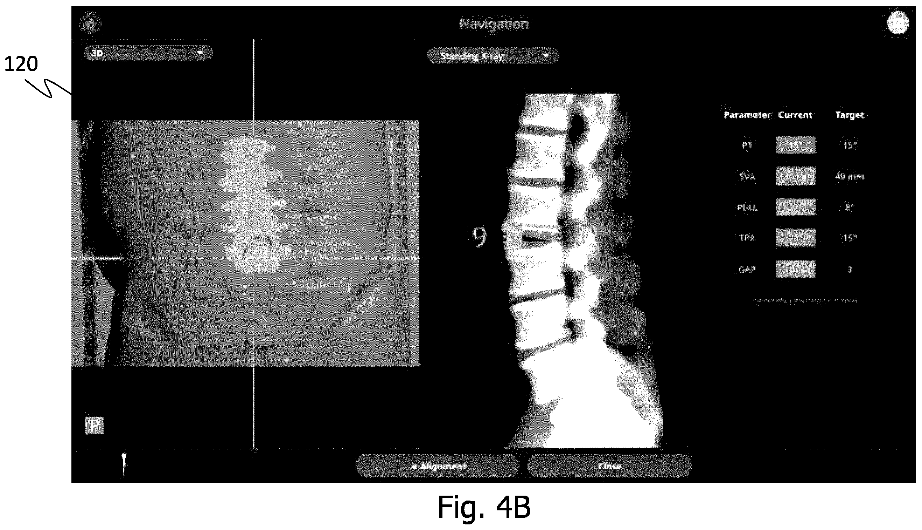

Further, Fig. 4B shows a 3D prediction image and a 2D prediction image indicating a current set of spinal balance parameters analogously to Fig. 4A and an associated set of target spinal balance parameters to be achieved when the indicated cage is implemented according the planning. Additionally, Fig. 4B shows the target position of the superior vertebra indicated by its inferior endplate. As a result, a user may easily derive from the shown values and the indicated target position whether a correct implant is implanted as planned.

In some examples, the method may comprise obtaining intra-operative image data and displaying the planned spinal cage implementation together with the intra-operative image data.

In some examples, tracking data indicative of poses of tracked vertebrae and/or a tracked instrument for inserting a spinal cage may be obtained and the intra-operative image may be adapted based on the tracking data allowing a real-time comparison between the current spinal balance parameters and the spinal balance parameters determined in the planning.

The features described in relation to the exemplary embodiments shown in the drawings can be readily combined to result in different embodiments. It is apparent, therefore, that the present disclosure may be varied in many ways. Such variations are not to be regarded as a departure from the scope of the invention as defined by the claims appended hereto.

The present technique will now be explained in other words. It is to be understood that the features disclosed in the following may be combined with the features discussed herein above and vice versa.

One objective of spinal fusion surgery is to fuse operative levels in a manner that restores a patient's balance, including alignment of the spinal curvature and restoration of disc height. Therefore, spinal fusion surgery commonly involves the insertion of interbody devices (cages), into the disc space. In conventional practice, alignment (e.g., part of an implementation) planning is frequently omitted, leaving the task solely to the surgeon's expertise. The suitability of, e.g., a shape/size (e.g., in combination with a position and orientation) of a spinal cage is commonly evaluated through trial fittings with (e.g., intra-operative) fluoroscopic imaging utilized to confirm a placement (e.g., as part of an implementation) of the spinal cage (e.g., based on intra-operative fluoroscopy images). Alternatively, existing software facilitating manual planning may be used by a surgeon. Omitting of planning may increase a health risk for a patient or decrease the mechanical stability of a spinal cage. Further, manual planning may be prone to human errors and thus put a high cognitive load on a surgeon and may only be performable by highly experienced surgeons.

Thus, there is need for an improved technique for planning of an implementation of one or more spinal cages.

In some variants of the third aspect explained herein, an automatic spinal alignment and interbody device (spinal cage) planning system 100 for spinal fusion surgery may be provided, for example, as extension of a navigation system. The system 100 may be configured for optimizing spinal curvature and balance in an upright position (e.g., according to steps 210 and 220 as described herein with reference to Fig. 2 / based on x-ray images in the standing position and respective optimal values for one or more spinal balance parameters). Moreover, the system 100 may be configured to provide a cage planning (e.g., according to step 230 as described herein with reference to Fig. 2 / implementation, e.g., shape, position and orientation planning of one or more spinal cages) supporting the computed, e.g., optimized spinal curvature and balance in the upright position. In some variants, the planning system 100 may additionally be configured for providing intra-operative feedback regarding the achievement of alignment goals and spinal cage placement.

A determined plan (e.g., for an implementation of a spinal cage) may be automatically generated and visualized after loading patient data allowing surgeons to modify and customize it to suit various surgical procedures. Furthermore, in cases where the pre-operative plan cannot be achieved, intra-operative re-optimization of interbody planning (e.g., re-calculating of an implementation plan based on obtained intra-operative image data) enables the calculation of the ideal interbody device (e.g., spinal cage) shape and pose to best accommodate the current spinal shape. The present technique may thus allow automatic planning within a navigation system directly after intra-operatively scanning the patient. With this capability the technique may provide a step towards evidenced based spine surgery and allows surgeons to optimize spinal parameters "on-the-fly".

For interbody (e.g., spinal cage implementation) planning, a 3D model may be used, whereas a 2D model derived from the LAT standing x-ray (e.g., a Lateral Prediction Image) may be sufficient to model aspects for lateral alignment. For lateral alignment automatic cage planning may (e.g., in some variants according to the first aspect described herein) be divided into: (a) Pre-operative calculation of an alignment and balancing target based on a patient's standing lateral full-spine x-ray (e.g., based on a lateral prediction image, for example according to steps 210 and 220 described herein) (b) Calculation of a 3D cage position and shape based on this target in a 3D prediction image, created by reformatting the operative levels of 3D imaging data such as a patient's computed tomography scan used for navigation.

The technique is not limited to this separation. For example, an anteroposterior full spine x-ray (e.g., scoliosis view) may be added for coronal adjustment; or the optimization may be performed (e.g., directly) on an 3D model that represents the standing patient condition (e.g., based on 3D patient images having adjusted poses as described herein).

For evaluating the balance and alignment of the spine, several parameters from medical literature may be considered.
- Spinal Alignment and Balancing Parameters, which are important for the health of the patient and the mechanical stability of the construct (e.g., vertebrae). Alignment targets describe the patient specific ideal shape of the vertebrae, whereas balance targets focus on Duboussets "cone of economy", to ensure that the head is located over the pelvis and to give the patient the least amount of energy expenditure to maintain an upright posture (e.g., SVA close to zero). Balance parameters are dependent on the alignment of the spine and on compensation mechanisms of the patient, like the Pelvic Tilt, Thoracic compensation, or knee flexion. Whereas alignment targets such as the lumbar lordosis (LL) are independent of these mechanisms and describe (e.g., local) alignment of the vertebrae in an asymptomatic individual.
- Disc Height: Restoring the optimal disc height is crucial for decompression and the stability as it decreases distractive and compressive forces on endplates.
- Anteroposterior (AP) Alignment: In addition, achieving alignment of vertebral bodies along the anterior-posterior axis is crucial for decompression and ensuring spinal stability during spinal fusion procedures.

Some variants according to the first aspect may comprise at least some of the following steps:
- obtaining a medical image, e.g., a 3D navigation image / 3D medical image data;
- obtaining a lateral, LAT, standing x-ray image (e.g., a 2D x-ray image);
- determining, based on image data, a 3D prediction image with optimized alignment, e.g., by:
   ∘ registering the 2D and 3D image data;
   ∘ creating a sagittal alignment target by optimizing an objective function, wherein the objective function may be iteratively optimized, and, e.g., by:
      ▪ creating a lateral prediction image (e.g., 2D prediction image) by modifying the standing x-ray image, e.g., by:
         - dividing the standing x-ray into image portions and changing a pose of the image portions;
         - applying a rotation (e.g., around a femur axis to simulate a pelvic tilt) to the modified medical image, e.g., to the x-ray comprising the changed poses; and
         - deriving patient specific parameters from the prediction images;
      ▪ computing the objective function based on patient specific information, e.g., like a T1 pelvic angle and/or a pelvic tilt;
   ∘ creating a 3D prediction image / modifying the 3D navigation image, e.g., by:
      ▪ aligning coronal and axial aspects;
- computing spinal cage positions (e.g., a spinal cage implementation comprising at least one of a position, an orientation, and a shape of a spinal cage) in a prediction image, e.g., by:
   ∘ obtaining alignment data;
   ∘ measuring disk space;
   ∘ optimizing a cage position and shape; and
   ∘ obtaining implant geometries of a given implant system (e.g., of a predetermined family of spinal cages)
- visualizing (e.g., of the computed implementation) and receiving optional user input; and
- receiving intra-operational image data and/or tracking data (e.g., indicative of at least one tracker associated with, e.g., one or more vertebrae or a tool for inserting a spinal cage) for determining movements (e.g., of one or more vertebrae and/or the tool) and triggering display thereof.

In the steps described above, some of the following data may be obtained and used:
- LAT Standing x-ray for creating the lateral prediction image;
- 3D Navigation Image for registration of the standing x-ray and optionally for deriving movement constraints. It may be used for creating the 3D prediction image on which cage planning may be performed;
- anatomical information for spinal parameters computation, depended on the chosen parameter set to be optimized, e.g.:
   ∘ vertebral levels included in the fusion (e.g., L5-L4);
   ∘ position of vertebral landmarks, at least the inferior and superior vertebrae of a fusion;
      ▪ e.g. superior/inferior anterior/posterior corners of the endplates;
      ▪ landmarks for spinal parameter computation, such as the center of C7 for SVA calculation;
   ∘ registration information for the standing X-ray such as corresponding endplate points in the 3D image and the standing x-ray;
   ∘ femur head positions for pelvic tilt simulation;
   ∘ a (e.g., full) vertebra segmentation in the 3D image to capture the exact surface and to perform geometrical computations regarding dexterity and collisions; furthermore, a bio-mechanical model may be used to enable highly accurate simulation of movement constraints and forces;
- Geometry information (e.g., the shape / width, length, height, lordotic angle) of cages to determine the ideal cage that fits into the optimized location (the ideal cage may be referred to as a "3D implant hull");
- Tracking data indicative of multiple vertebrae poses to enable intra-operative optimization of cage planning.

In more detail, the 3D (medical) imaging data such as a patient's computed tomography scan may be obtained by the system 100. For example, the obtained 3D imaging data may be or comprise a 3D image based on which a navigated spine surgery is performed. Depending on the surgical approach the 3D imaging data may capture the operative levels of the patient in a position that differs from the standing position, e.g. the patient being in the prone or lateral position.

Vertebrae may be segmented automatically, and landmarks may be identified for registering a standing X-ray.

In addition to the 3D imaging data, a standing X-ray may be loaded into the system 100 (e.g., obtained by the system 100).

Landmarks for registration and spinal parameter calculation including endplate positions may be identified.

Segmentation and/or landmark identification in 2D and 3D imaging data may be derived automatically with a Deep Learning network.

An optimal vertebral repositioning may be simulated to obtain a post-operative prediction image estimating the spinal shape in the standing posture.

After calculation of the alignment goal, the exact size and position of the interbody device may be computed in a 3D prediction image.

The mapping between the, e.g., 3D navigation image and the LAT standing x-ray image may be derived by a 3D to 2D registration process. Establishing the mapping may allow length measurements in the standing x-ray without additional reference objects. The mapping may be needed for transferring a computed cage position from the lateral image to the 3D image and for transferring measurements of vertebral positions in a navigation system to the standing x-ray. The latter may be needed for intra-operative adjustment of a cage plan in cases where the pre-op plan could not be achieved.

There are several well-known methods known for 3D to 2D registration. A simple point-to-point registration may be applied under the assumption that the lateral standing x-ray is perfectly lateral oriented. Anterior and posterior endplate points may be chosen as landmarks for establishing the point-to-point registration. Determining the registration may comprise projecting landmarks of the 3D navigation image to the sagittal plane and deriving a 2D registration matrix that transforms corresponding points from the sagittal projection to the standing lateral x-ray with a known landmark transformation algorithm. As landmarks, a pair of anterior and posterior points of the superior endplate of the inferior reference vertebrae may be used.

After creating an aligned 2D prediction image, resulting positions may be transferred back to the 3D navigation image by applying the inverse transformation. By incorporating the initial transformation in the lateral image into the transformation chain, also intra-operatively measured 3D positions of vertebrae may be reformatted to the standing x-ray. This allows estimation of a standing x-ray in an upright position of the patient.

Calculate the sagittal alignment and balancing target may include compensation mechanisms like Pelvic Tilt, disc height restauration and anterior-posterior alignment by optimizing the objective function. The optimization may be described as: ${p}_{optimal} = {argmin}_{θ} f \left(p\right)$ (2) with p describing the position of the vertebrae in the lateral prediction image, θ describing the movement constraints of the vertebrae, and f being an objective function that is minimized to get an optimal alignment pₒₚₜᵢₘₐₗ. The optimal alignment may describe the position of the vertebrae of the aligned and balanced spine in the lateral prediction image including compensation mechanisms.

To optimize the objective function, iterative metaheuristics for global optimization may be used such as multi-level gradient decent. Hence, various simulated positions of the vertebrae that meet specified movement constraints, may be evaluated by the objective function, and the best solution may be chosen. Thus, after several iterations the alignment may be approximated that optimizes the objective function. As lateral alignment and balancing parameters may need to be measured in the upright standing position, potential vertebral movements of the instrumented levels may be simulated by modifying the standing x-ray image to calculate a lateral (2D) prediction image (as shown in Fig. 3B).

Figs. 3A and 3B show a potential modification of a standing X-ray by introducing a wedge as a result of a transformation of the superior image (part) in respect to a modified vertebral level (e.g. L5-L4). Therefore, the (2D prediction) image may be divided into superior and inferior parts, both may be treated as rigid.

To achieve the optimal alignment the simulation (the computer-implemented method according to the first aspect as described herein) may comprise evaluating a fusion of different levels, such as a L5-L4 fusion, or an L5-L3 fusion. Each time, a prediction image may be created by introducing at least one wedge.

Dependent on the use case, the simulation may be performed just for a fixed level such as L5-L4, for any adjacent pair of levels, or for multi-level fusions:
- Fixed 2-level fusion
   ∘ This option involves a predefined fusion of two vertebrae, such as L5-L4, to determine one optimal wedge between the specified vertebrae.
- Variable 2-level fusion
   ∘ In this variant, the simulation is applied to all adjacent vertebrae in a given range (e.g., S1 to L1), resulting in one optimal wedge between any of the adjacent levels (S1-L5, L5-L4, L4-L3, L3-L2 or L2-L1).
- Variable multi-level fusion
   ∘ E.g. all 3-level fusions of adjacent vertebrae starting with S1 as the inferior vertebrae and ending with L1 as superior vertebrae, namely the S1-L4, L5-L3, L4-L2 and L3-L1 fusion.

The selection of instrumented levels may be determined automatically based on the simulation of the different (level fusion) approaches and the resulting values of the objective function. In detail, the simulation process may start by evaluating all 2-level fusions, followed by the assessment of higher-level fusions. Generally, a fusion of more levels tends to minimize the gap between the ideal and simulated values. If the improvement resulting from adding another level falls below a predefined threshold, the optimal outcome from the fusion with fewer levels my be selected.

This systematic approach may allow for comprehensive exploration of fusion scenarios and facilitates the identification of the most suitable wedge configuration to optimize spinal alignment and biomechanical stability.

As fusing more levels, may mean a more invasive procedure and may restrict mobility of the patient, determining an appropriate threshold deciding whether it is beneficial to extend the surgery by one level may be critical. Thus, the result of a chosen threshold may be presented (visualized / displayed) as suggestion to a user, e.g., a surgeon, but allowing the surgeon to specify the number of levels and constrain the optimization accordingly.

For deriving a lateral (2D) prediction image, an obtained (2D medical) image may be divided into image portions which are transformed, e.g., by:
- dividing the image into image portions, e.g., by splitting the image at the level, where the fusion is simulated, for example by choose cutting line through the anterior and posterior endplate points of the inferior endplate of the superior vertebrae; and
- transforming superior part of the image, e.g., by choosing a reference point in the superior image, e.g. the posterior, inferior endplate point of the superior vertebra, translating the superior part of the image including the reference point, and rotating the superior part of the image around the resulting reference point.

The transformation applied to the superior image part may consist of 3 degrees of freedom: AP-translation, cranial-caudal translation (disc height restauration) and rotation (lordosis/kyphosis increase).

For each fusion level a respective transformation may be computed and applied to the superior part of the standing x-ray (2D medical image), to create a lateral (2D) prediction image, creating a so called "wedge".

For simulating a pelvic tilt, the whole prediction image may be rotated around the femur head. If the lateral image is not perfectly sagittal two femur heads might be visible and the center of both may be chosen as rotation point. Other compensations mechanisms may be included to model wedges in the thoracic region.

Patient specific parameters may be obtained, which may be used or even necessary for computing spinal parameters and evaluating their values, as follows:
- measured in the imaging data (e.g. pelvic incidence),
- derived from the DICOM data (e.g. gender, age), and
- optionally provided to the system 100 (e.g. BMI).

After creating a lateral prediction image to be evaluated, the objective function may be computed based on measurements in the prediction image. The objective function may quantify the deviation from ideal values across a defined set of parameters to provide a comprehensive assessment of spinal health and function, guiding clinical decision-making and therapeutic interventions aimed at optimizing spinal alignment and biomechanical stability.

The objective function may be calculated as a weighted sum of parameters pᵢ as defined in equation (1) above. The objective function may depend on the position of the vertebrae, e.g., based on the vertebra position, the optimal values may change.

Examples for ideal values derived from the GAP score are:
- $SS = PI ⋅ 0.59 + 9$
- $LL = PI ⋅ 0.62 + 29$
- $LDI = L 4 S 1 Lordosis / L 1 S 1 Lordosis ⋅ 100 % = 65 %$
- $GT = 0.48 ⋅ PI - 15$

Additional aspects focusing on upper parts of a spine may be included as well as thoracic compensation mechanisms.

For each of the parameters pᵢ an ideal value may be provided, a normative range, a range of moderately misaligned state and a severely misaligned state. Constraints may be included for the optimization to capture potentially possible values, and to for example exclude a Pelvic Tilts outside the range of -5 to 40°. Additional constraints may be derived like collision between vertebrae.

Further, excessively large cage heights may result in supraphysiologic interbody space restoration and potentially predispose to complications. Thus, deviations from a normative range may be included into the objective function to restore an appropriate disc height and to integrate limitations for stretching in cranial-caudal direction.

For example, a gender specific range for the anterior and posterior disc height of each level such as a L4/5 anterior disc height may be defined as 10.9 +/- 2.1 mm and a posterior disc height of 6.1+1.4 mm for men. A range of the defined mean +/-2 times standard deviation may be used as constraints for the optimization.

By constructing a parallel line to a superior endplate of an inferior vertebra which includes an endpoint of the superior endplate, an anterior respectively posterior disc height can be calculated as the distance between both lines.

Further, an anterior and posterior AP translation may be included into the objective function. For example, an ideal of 0 mm and a normative range of e.g. 2 mm may be assumed.

The optimized sagittal condition may be transferred to a 3D prediction image by applying the inverse of a 3D to 2D registration resulting in an 3D image, that is modified in respect to the sagittal aspect.

A coronal and axial alignment may be applied to the (modified) 3D prediction image. In general, the target is 0° for the Cobb angle (straight spine in AP view) and the axial rotation.

To align the AP aspect, the superior endplate of the superior vertebra and the inferior endplate of the inferior vertebrae may be projected to the AP plane. Ideally these endplates should be parallel resulting in an AP Cobb angle of 0°. To create the 3D prediction model, the superior part of the image may be rotated around the center of the superior endplate to make both endplates parallel.

The same procedure may be applied to achieve a 0° axial angle: The superior part of the 3D image may be rotated around the center of a foramen (of a vertebra) to achieve a 0° axial angle.

Based on the 3D positions of the instrumented (fused / modified) levels an automatic cage planning may be performed.

Further, following the workflow of pre-operatively generating the alignment target (e.g., determining a prediction image), a cage planning algorithm may use the target defined by the 3D prediction image as input. However, if, e.g., a surgeon cannot achieve the alignment goal, intraoperatively measured positions of the vertebrae may be used as input instead.

To determine constraints for the cage shape, an exact 3D disc space may be measured, e.g. as a convex hull between two adjacent endplates of segmented vertebrae. The objective may be to identify an ideal cage geometry that fits into the (optimized) disc space while providing contact to the endplates. Like mentioned above, the optimized 3D prediction image may be preferably used as input and just in cases where the surgeon triggers an intra-operative cage re-optimization, intra-operatively measured vertebral positions may be used as input.

A normative volume for optimal cage positions may be computed, denoted as "3D implant hull". The 3D implant hull may be a sub-volume of a disc space and may define a region where a cage should optimally be located. For different planned surgical approaches such as Transforaminal Lumbar Interbody Fusion, TLIF, or Posterior Lumbar Interbody Fusion, PLIF, different areas may be defined.

For example, a simple bounding region may be defined and an intersection of this region with a disc space may be defined as "3D implant hull" or forbidden zones may additionally be subtracted from it.

In detail, first a bounding region proportional to the geometry of the calculated disc space may be calculated. Then, an anterior boundary may be computed as the volume located anterior to the calculated disc space. Thus, the bounding region may be further limited to finally define the 3D implant hull.

Choosing the bounding region may primarily involve deciding an anterior-posterior placement of the cage, whereas the lordotic angle, the cage height and length may be derived from measurements of the 3D disc space. The lordotic angle and the cage height highly correlate with the sagittal angle and the disc height (the wedge) between the adjacent vertebrae, while the cage length may be calculated by measured proportions of the disc space. For example, in a TLIF approach, a cage may be placed maximally anterior while maximizing the length of the cage. In a lateral approach, a cage may be placed in the middle of a vertebral body while maximizing the length of the cage.

Similarly, for alternative approaches such as oblique TLIF, Anterior-To-Psoas, ATP, or PLIF, an ideal anterior-posterior placement may be defined based on the proportions of the vertebral anatomy, and the exact lordotic angle as well as the cage height may be derived from measurements of the 3D disc space (in the 3D prediction image).

After defining the 3D implant hull, a specific cage geometry (e.g., a Computer Aided Design, CAD, file) from a selected cage family may be chosen which fits best to the 3D implant hull.

In an alternative approach, the first step of computing an 3D implant hull and later selecting the best fitting cage may be omitted; and cages of a specific family might be already included in the optimization procedure. This may be implemented by one of the following options: a) possible alignments in the optimization procedure may be limited to ones that are achievable with a given family of cages; or b) a metric is included in the objective function describing the gap between the simulated alignment and the alignment that can be achieved with a given cage.

The optimization algorithm may be triggered after a lateral image has been loaded and necessary landmarks have been identified automatically or manually and confirmed by a surgeon. The resulting alignment goal may be visualized, as well as the planned cage. Further, optimal fusion levels may be visualized, e.g., as suggestion in the lateral (2D) prediction image.

A surgeon may be provided with the option to modify the wedge(s) and to re-run the optimization with the following two options:
a) Fixed Level Selection:
   Surgeons may choose a specific fusion level and potentially utilize an "optimize" button to optimize the configuration for the selected level.
b) Variable Level Selection:
   Surgeons may have the flexibility to select the number of fusion levels and reoptimize by using a "Calculate optimal n-level fusion" function, specifying the desired number n of levels.

Further options for changing the alignment target may be given:
- the wedge angel(s);
- the pelvic tilt; and
- the disc height(s).

For the cage, the surgeon may be able to adjust the:
- surgical approach such as traditional TLIF or PLIF;
- cage position such as the anterior-posterior placement of the cage(s); and
- cage shape such as the length.

Upon receiving a confirmation of the outlined planning, both, the cage position and the alignment target may be visualized in relation to an anatomy in a navigation view, as well as information derived from it, like the disc height as shown in Fig. 4B.

Up to this point, the described steps do not require any tracking information and may be pre-operatively computed and visualized, functioning as a standalone system 100 for cage and alignment planning (planning of a spinal cage implementation according to the first aspect described herein). Further integration of tracking information may enable intraoperative guidance for a surgeon, allowing real-time assessment of alignment goal achievement and optimal cage placement.

By tracking and visualizing a cage attached to a cage inserter (e.g., a tool configured for receiving and inserting a spinal cage) or information derived from it, a position of a navigated cage may be visually compared to a position of a planned cage. The visualization may incorporate Augmented Reality to visualize the cage position directly in-situ.

Inserting the cage and aligning the spine changes the anatomy, so it differs from a medical image used for navigation (e.g., the 3D medical image). It may thus be beneficial to display the vertebral movement during disc spreading and cage insertion in a navigation system. It may also be beneficial to show the computed lateral (2D) prediction image for the standing posture and the computed spinal parameters in real-time during the intervention.

The benefits of visualizing multiple vertebrae during the intervention may extend beyond visualization. The ability to localize vertebral movements may enable triggering of a re-planning process for an optimal cage that conforms to the current spine shape. Instead of a pre-operatively created prediction image (2D or 3D prediction images), the current position of the vertebrae may serve as input to initiate an automatic cage planning process as described herein.

## Claims

1. A computer-implemented method (200) for automatically planning an implementation of one or more spinal cages, comprising:
obtaining (210) image data including a 2D medical image comprising representations of a plurality of vertebrae of a patient's body in a standing body posture;
determining (220) a 2D prediction image comprising representations of the plurality of vertebrae of the patient's body in the standing body posture, wherein determining the 2D prediction image comprises modifying the 2D medical image such that at least one spinal balance parameter derivable from the 2D prediction image fulfills a predefined criterion; and
determining (230), based on the 2D prediction image, an implementation of one or more spinal cages yielding the poses of the plurality of vertebrae as represented by the 2D prediction image, wherein
determining (230) the implementation of the one or more spinal cages comprises transferring the poses of the vertebrae as represented by the 2D prediction image onto vertebrae represented by a 3D patient image, and determining the implementation based on the vertebrae represented by the 3D patient image having the transferred poses, **characterized in that**
the transferred poses of vertebrae of the 3D patient image are further adjusted, in one or more planes differing from an imaging plane of the 2D medical image, before determining the implementation of the one or more spinal cages based on the vertebrae represented by the 3D patient image that have the so-adjusted poses.

2. The method (200) of claim 1, further comprising determining a registration for registering the 2D medical image with the 3D patient image, wherein the poses of the vertebrae as represented by the 2D prediction image are transferred onto vertebrae represented by the 3D patient image based on the registration of the 2D medical image with the 3D patient image.

3. The method (200) of claim 1 or 2, wherein
the further adjustment of the transferred poses comprises at least one of a cobb angle alignment and an axial alignment of the vertebrae.

4. The method (200) of any preceding claim, wherein determining (220) the 2D prediction image comprises simulating a fusion of different vertebra levels; and, optionally, wherein
i) the fusion of different vertebra levels comprises a fusion of at least three vertebra levels; and/or
ii) the fusion of different vertebra levels is simulated based on a predefined maximum number of vertebra levels to be fused.

5. The method (200) of claim 4, further comprising:
outputting a respective optimal fusion result for each number of vertebra levels to be fused;
receiving user-input selecting one of the respective optimal fusion results; and
determining the implementation of the one or more spinal cages based on the selected respective optimal fusion result.

6. The method (200) of claim 4 or 5, wherein simulating the fusion of different vertebra levels comprises introducing at least one virtual wedge between vertebrae that are simulated to be fused together, for example between vertebrae represented by the 2D medical image or vertebrae represented by a or the 3D patient image; and, optionally, wherein
introducing a wedge comprises splitting the obtained 2D medical image into at least two parts and moving the at least two parts relative to each other, optionally within 3 degrees of freedom.

7. The method (200) of any preceding claim, wherein the at least one spinal balance parameter is indicative of at least one of a pelvic tilt, thoracic compensation, knee flexion, disc height restauration and anterior-posterior alignment.

8. The method (200) of any preceding claim, further comprising obtaining one or more patient specific parameters and deriving the at least one spinal balance parameter based at least in part on the one or more patient specific parameters.

9. The method (200) of any preceding claim, wherein multiple spinal balance parameters are derived from the 2D prediction image and the implementation of one or more spinal cages is determined based on a combination of at least some of the multiple spinal balance parameters, wherein, optionally, the combination comprises determining a weighted sum of the derived multiple spinal balance parameters.

10. The method (200) of claim 9, wherein deriving the multiple spinal balance parameters from the 2D prediction image comprises determining multiple spinal balance parameters sets and selecting one of the sets for determining the implementation based on at least one optimization criterium.

11. The method (200) of any preceding claim, wherein determining (230) the implementation of the one or more spinal cages comprises at least one of determining a position and orientation of the one or more spinal cages and determining a respective shape for each of the one or more spinal cages based at least in part on the 2D prediction image or the 3D patient image comprising the transferred poses.

12. The method (200) of claim 11, further comprising selecting, based on the determined respective shapes, a spinal cage from a family of predetermined spinal cages matching the determined respective shape the best.

13. The method (200) of any preceding claim, wherein determining (230) the implementation of the one or more spinal cages comprises:
determining, based at least in part on the 2D prediction image or the 3D patient image comprising the transferred poses, a region within a space between two adjusted vertebrae to place one of the one or more spinal cages in; and
determining a position for said spinal cage within the determined region based at least in part on the 2D prediction image.

14. The method (200) of claims 12 and 13, further comprising triggering display of a visualization indicating the determined spinal cage implementation together with the selected spinal cage and, optionally, indicating optimal fusion levels of the plurality of vertebrae.

15. The method (200) of claim 14, wherein the visualization comprises at least one of:
i) a visualization in the 2D prediction image;
ii) a visualization in the 3D patient image comprising the transferred poses; and
iii) a visualization of the selected cage attached to a cage insertion instrument.

16. The method (200) of claim 14 or 15, further comprising:
receiving user-input adjusting at least one of the visualized spinal cage implementation and the selected spinal cage; and
re-calculating the spinal cage implementation and the spinal cage shape based on the user-input; and, optionally, wherein
the user-input further indicates specific levels of vertebrae to be fused or a specific number of vertebra levels to be fused.

17. A computer program product, comprising instructions that, when executed on at least one processor, cause the at least one processor to carry out any of the methods of claims 1 to 16.

18. A planning system (100) comprising at least one processor configured to carry out the method (200) of any one of claims 1 to 16.

19. The planning system (100) of claim 18, further comprising at least one of the following entities:
a medical imaging device (130) configured to generate at last one of the 2D medical image and the 3D patient image;
a user input device configured to receive the user-input;
a display device (120) configured to display the visualization.

## Patentansprüche

1. Computer-implementiertes Verfahren (200) für das automische Planen einer Implementierung von einem oder mehreren Wirbelsäulen-Cages, umfassend:
Erhalten (210) von Bilddaten, die ein 2D medizinisches Bild beinhalten, das Repräsentationen einer Vielzahl von Wirbeln eines Patientenkörpers in einer stehenden Körperhaltung umfasst;
Bestimmen (220) eines 2D Prognosebildes umfassend Repräsentationen der Vielzahl von Wirbeln des Patientenkörpers in der stehenden Körperhaltung, wobei das Bestimmen des 2D Prognosebildes ein Modifizieren des 2D medizinischen Bildes derart, dass mindestens ein aus dem 2D Prognosebild ableitbarer Wirbelsäulenbalanceparameter ein vordefiniertes Kriterium erfüllt, umfasst; und
Bestimmen (230), basierend auf dem 2D Prognosebild, einer Implementierung von einem oder mehreren Wirbelsäulen-Cages, welche die Posen der Vielzahl von Wirbeln, so wie repräsentiert durch das 2D Prognosebild, ergeben, wobei
Bestimmen (230) der Implementierung des einen oder der mehreren Wirbelsäulen-Cages ein Übertragen der Posen der Wirbel, so wie repräsentiert durch das 2D Prognosebild, auf durch ein 3D Patientenbild repräsentierte Wirbel und ein Bestimmen der Implementierung basierend auf den durch das 3D Patientenbild repräsentierten Wirbeln, welche die übertragenen Posen haben, umfasst, **dadurch gekennzeichnet, dass**
die übertragenen Posen der Wirbel des 3D Patientenbildes in einer oder mehreren Ebenen, die sich von einer Bildebene des 2D medizinischen Bildes unterscheiden, weiter angepasst werden, bevor die Implementierung des einen oder der mehreren Wirbelsäulen-Cages basierend auf den durch das 3D Patientenbild repräsentierten Wirbeln, welche die so angepassten Posen haben, bestimmt wird.

2. Verfahren (200) nach Anspruch 1, ferner umfassend Bestimmen einer Registrierung zum Registrieren des 2D medizinischen Bildes mit dem 3D Patientenbild, wobei die Wirbelposen wie von dem 2D Prognosebild repräsentiert, übertragen werden auf die durch das 3D Patientenbild repräsentierten Wirbeln, basierend auf der Registrierung von dem 2D medizinischem Bild mit dem 3D Patentenbild.

3. Verfahren (200) nach Anspruch 1 oder 2, wobei
die weitere Anpassung der übertragenen Posen mindestens eines aus einer Cobb-Winkel-Ausrichtung und einer axialen Ausrichtung der Wirbel umfasst.

4. Verfahren (200) nach einem der vorangegangenen Ansprüche, wobei Bestimmen (220) des 2D Prognosebildes ein Simulieren einer Fusion von verschiedenen Wirbellevels umfasst; und wobei, optional
i) die Fusion verschiedener Wirbellevels eine Fusion von mindestens drei Wirbellevels umfasst; und/oder
ii) die Fusion verschiedener Wirbellevels basierend auf einer vordefinierten maximalen Anzahl an zu fusionierenden Wirbellevels simuliert ist.

5. Verfahren (200) nach Anspruch 4, ferner umfassend:
Ausgeben eines jeweils optimalen Fusionsergebnisses für jede Nummer der zu fusionierenden Wirbellevels;
Erhalten einer Nutzereingabe, die eines der jeweils optimalen Fusionsergebnisse auswählt; und
Bestimmen der Implementierung des einen oder der mehreren Wirbelsäulen-Cages basierend auf dem jeweils ausgewählten optimalen Fusionsergebnis.

6. Verfahren (200) nach Anspruch 4 oder 5, wobei ein Simulieren der Fusion von verschiedenen Wirbellevels ein Einfügen mindestens eines virtuellen Keils umfast zwischen Wirbel, die zum miteinander fusioniert werden simuliert werden, zum Beispiel zwischen durch das 2D medizinische Bild oder durch ein oder das 3D Patientenbild repräsentierte Wirbel, umfasst; und wobei, optional,
Einführen eines Keils ein Aufteilen des erhaltenen 2D medizinischen Bildes in mindestens zwei Teile und Bewegen der mindestens 2 Teile relativ zueinander, optional mit 3 Freiheitsgraden, umfasst.

7. Verfahren (200) nach einem der vorangegangenen Ansprüche, wobei der mindestens eine Wirbelsäulenbalanceparameter indikativ ist für mindestens eines von: Beckenneigung, Brustwirbelbereichkompensation, Kniebeugung, Bandscheibenhöhewiederherstellung und anterior-posterioren Ausrichtung.

8. Verfahren (200) nach einem der vorangegangenen Ansprüche, ferner umfassend Erhalten eines oder mehrerer patientenspezifischen Parameter und Ableiten des mindestens einen Wirbelsäulenbalanceparameters, der zumindest teilweise basierend ist auf dem einen oder mehreren patientenspezifischen Parameter(n).

9. Verfahren (200) nach einem der vorangegangenen Ansprüche, wobei mehrere Wirbelsäulenbalanceparameter abgeleitet sind von dem 2D Prognosebild und die Implementierung eines oder mehrerer Wirbelsäulen-Cages auf der Kombination von mindestens einigen der mehreren Wirbelsäulenbalanceparameter basiert, wobei optional die Kombination Bestimmen einer gewichteten Summe von den abgeleiteten mehreren Wirbelsäulenbalanceparametern umfasst.

10. Verfahren (200) nach Anspruch 9, wobei Ableiten der mehreren Wirbelsäulenbalanceparameter von dem 2D Prognosebild Bestimmen mehrerer Wirbelsäulenbalanceparametersets und Auswählen eines der Sets zum Bestimmen der Implementierung basierend auf mindestens einem Optimierungskriterium, umfasst.

11. Verfahren (200) nach einem der vorangegangenen Ansprüche, wobei Bestimmen (230) der Implementierung des einen oder der mehreren Wirbelsäulen-Cages mindestens eines von Bestimmen einer Position und Orientierung des einen oder der mehreren Wirbelsäulen-Cages und Bestimmen einer jeweiligen Form für jede des einen oder der mehreren Wirbelsäulen-Cages, mindestens teilweise basierend auf dem 2D Prognosebild oder dem 3D Patientenbild, das die übertragenen Posen umfasst, umfasst.

12. Verfahren (200) nach Anspruch 11, ferner umfassend Auswählen basierend auf den jeweiligen bestimmten Formen, eines am besten zur jeweils bestimmten Form passenden Wirbelsäulen-Cages aus einer Familie vorbestimmter Wirbelsäulen-Cages.

13. Verfahren (200) nach einem der vorangegangenen Ansprüche, wobei Bestimmen (230) der Implementierung des einen oder der mehreren Wirbelsäulen-Cages umfasst:
Bestimmen, mindestens teilweise basierend auf dem 2D Prognosebild oder dem die übertragenen Posen umfassenden 3D Patientenbild, eines Bereiches innerhalb eines Raumes zwischen zwei angepassten Wirbeln, in dem einer des einen oder der mehreren Wirbelsäulen-Cages platziert werden; und
Bestimmen einer Position für besagten Wirbelsäulen-Cage in dem bestimmten Bereich mindestens teilweise basierend auf dem 2D Prognosebild.

14. Verfahren (200) nach Anspruch 12 und 13, ferner umfassend Auslösen einer Anzeige einer Visualisierung, die die bestimmte Wirbelsäulen-Cage-Implementierung zusammen mit dem ausgewählten Wirbelsäulen-Cage angibt, und, optional, optimale Fusionslevels der Vielzahl an Wirbeln, angibt.

15. Verfahren (200) nach Anspruch 14, wobei die Visualisierung mindestens eines umfasst von:
i) einer Visualisierung in dem 2D Prognosebild;
ii) einer Visualisierung in dem 3D Patientenbild, das die übertragenen Posen umfasst;
und
iii) einer Visualisierung des ausgewählten, an einem Cage-Einführungsinstrument befestigten Cages.

16. Verfahren (200) nach Anspruch 14 oder 15, ferner umfassend:
Erhalten von Nutzereingabe, die mindestens eine der visualisierten Wirbelsäulen-Cage-Implementierung und des ausgewählten Wirbelsäulen-Cage anpasst; und
Neuberechnen der Wirbelsäulen-Cage-Implementierung und der Form des Wirbelsäulen-Cages basierend auf der Nutzereingabe; und wobei, optional,
die Nutzereingabe ferner spezifische Level zu fusionierender Wirbel oder eine spezifische Zahl an zu fusionierenden Wirbellevels angibt.

17. Computerprogrammprodukt, das Anweisungen umfasst, die, wenn auf mindestens einem Prozessor ausgeführt, den mindestens einen Prozessor dazu veranlassen eines der Verfahren aus Ansprüchen 1 bis 16 auszuführen.

18. Planungssystem (100), das mindestens einen Prozessor umfasst, der dazu konfiguriert ist, das Verfahren (200) aus einem der Ansprüche 1 bis 16 auszuführen.

19. Planungssystem (100) nach Anspruch 18, ferner umfassend mindestens eine der folgenden Entitäten:
ein medizinisches Bildgebungsgerät (130), das dazu konfiguriert ist, mindestens eines des 2D medizinisches Bildes und des 3D Patientenbildes zu generieren;
ein Nutzer-Eingabegerät, das dazu konfiguriert ist, Nutzer-Eingabe zu erhalten;
ein Anzeigegerät (120), das dazu konfiguriert ist, die Visualisierung anzuzeigen.

## Revendications

1. Procédé mis en œuvre par ordinateur (200) pour planifier automatiquement une mise en place d'une ou plusieurs cages intersomatiques, comprenant:
l'acquisition (210) de données d'image comprenant une image médicale 2D représentant une pluralité de vertèbres du corps d'un patient debout;
la détermination (220) d'une image de prédiction 2D comprenant des représentations de la pluralité de vertèbres du corps du patient debout, la détermination de l'image de prédiction 2D consistant à modifier l'image médicale 2D de telle sorte qu'au moins un paramètre d'équilibre rachidien pouvant être obtenu à partir de l'image de prédiction 2D réponde à un critère prédéfini; et
la détermination (230), à partir de l'image de prédiction 2D, d'une mise en place d'une ou plusieurs cages intersomatiques permettant d'obtenir les positions de la pluralité de vertèbres telles qu'elles sont représentées par l'image de prédiction 2D, dans lequel
la détermination (230) de la mise en place d'une ou plusieurs cages intersomatiques consiste à transférer les positions des vertèbres représentées par l'image de prédiction en 2D, vers les vertèbres représentées par une image 3D du patient, et la détermination de la mise en place sur la base des vertèbres représentées par l'image 3D du patient présentant les positions transférées, **caractérisé en ce que**
les positions transférées des vertèbres de l'image 3D du patient sont ensuite ajustées, dans un ou plusieurs plans différents du plan d'imagerie de l'image médicale 2D, avant de déterminer la mise en place d'une ou plusieurs cages intersomatiques sur la base des vertèbres représentées par l'image 3D du patient, dont les positions ont ainsi été ajustées.

2. Procédé (200) selon la revendication 1, comprenant en outre la détermination d'un recalage permettant de mettre en correspondance l'image médicale 2D avec l'image 3D du patient, dans lequel les positions des vertèbres représentées par l'image de prédiction 2D sont superposées sur les vertèbres représentées par l'image 3D du patient sur la base du recalage de l'image médicale 2D par rapport à l'image 3D du patient.

3. Procédé (200) selon la revendication 1 ou 2, dans lequel
l'ajustement supplémentaire des positions transférées comprend au moins l'un parmi un alignement selon l'angle de Cobb ou un alignement axial des vertèbres.

4. Procédé (200) selon l'une quelconque revendication précédente, dans lequel la détermination (220) de l'image de prédiction en 2D comprend la simulation d'une fusion de différents niveaux vertébraux; et, éventuellement, dans lequel
i) la fusion de différents niveaux vertébraux comprend la fusion d'au moins trois niveaux vertébraux; et/ou
ii) la fusion de différents niveaux vertébraux est simulée en fonction d'un nombre maximal prédéfini de niveaux vertébraux à fusionner.

5. Procédé (200) selon la revendication 4, comprenant en outre:
la sortie d'un résultat de fusion optimal correspondant à chaque nombre de niveaux vertébraux à fusionner;
la réception d'une entrée utilisateur permettant de sélectionner l'un des résultats de fusion optimaux correspondants; et
la détermination de la mise en place d'une ou plusieurs cages intersomatiques en fonction du résultat de fusion optimal respectif sélectionné.

6. Procédé (200) selon la revendication 4 ou 5, dans lequel la simulation de la fusion de différents niveaux vertébraux comprend l'introduction d'au moins une cale virtuelle entre les vertèbres dont on simule la fusion, par exemple entre les vertèbres représentées par l'image médicale 2D ou les vertèbres représentées par une image ou l'image 3D du patient; et, éventuellement, dans lequel
l'introduction d'une cale consiste à diviser l'image médicale 2D obtenue en au moins deux parties et à déplacer les au moins deux parties l'une par rapport à l'autre, éventuellement selon trois degrés de liberté.

7. Procédé (200) selon l'une quelconque revendication précédente, dans lequel l'au moins un paramètre d'équilibre rachidien indique au moins l'un parmi l'inclinaison pelvienne, la compensation thoracique, la flexion du genou, la restauration de la hauteur discale et l'alignement antéro-postérieur.

8. Procédé (200) selon l'une quelconque revendication précédente, comprenant en outre l'obtention d'un ou plusieurs paramètres spécifiques au patient et le calcul d'au moins un paramètre d'équilibre rachidien sur la base, au moins en partie, du ou des paramètres spécifiques au patient.

9. Procédé (200) selon l'une quelconque revendication précédente, dans lequel plusieurs paramètres d'équilibre rachidien sont calculés à partir de l'image de prédiction 2D et la mise en place d'une ou plusieurs cages intersomatiques est déterminée sur la base d'une combinaison d'au moins certains des multiples paramètres d'équilibre rachidien, dans lequel, éventuellement, la combinaison comprenant la détermination d'une somme pondérée des paramètres d'équilibre rachidien calculés.

10. Procédé (200) selon la revendication 9, dans lequel le calcul des multiples paramètres d'équilibre rachidien à partir de l'image de prédiction 2D comprend la détermination de multiples ensembles de paramètres d'équilibre rachidien et la sélection de l'un des ensembles pour déterminer la mise en place sur la base d'au moins un critère d'optimisation.

11. Procédé (200) selon l'une quelconque revendication précédente, dans lequel la détermination (230) de la mise en place d'une ou plusieurs cages intersomatiques comprend au moins l'une parmi la détermination d'une position et une orientation de la ou des cages intersomatiques, et la détermination d'une forme respective pour chacune de la ou des cages intersomatiques sur la base, au moins en partie, de l'image de prédiction 2D ou de l'image 3D du patient comprenant les positions transférées.

12. Procédé (200) selon la revendication 11, comprenant en outre la sélection, sur la base des formes respectives déterminées, d'une cage intersomatiques parmi une famille de cages intersomatiques prédéterminées correspondant le mieux à la forme respective déterminée.

13. Procédé (200) selon l'une quelconque revendication précédente, dans lequel la détermination (230) de la mise en place d'une ou plusieurs cages intersomatiques comprend:
la détermination, sur la base, au moins en partie, de l'image de prédiction 2D ou sur l'image 3D du patient comprenant les positions transférées, d'une zone située dans l'espace entre deux vertèbres ajustées, dans laquelle placer l'une parmi la ou les cages intersomatiques; et
la détermination d'une position pour ladite cage intersomatique dans la zone déterminée, sur la base, au moins en partie, de l'image de prédiction 2D.

14. Procédé (200) selon les revendications 12 et 13, comprenant en outre le déclenchement de l'affichage d'une visualisation indiquant la mise en place déterminée de la cage intersomatique conjointement avec la cage intersomatique sélectionnée et, éventuellement, indiquant les niveaux de fusion optimaux de la pluralité de vertèbres.

15. Procédé (200) selon la revendication 14, dans lequel la visualisation comprend au moins l'une parmi:
i) une visualisation dans l'image de prédiction 2D;
ii) une visualisation dans l'image 3D du patient comprenant les positions transférées; et
iii) une visualisation de la cage sélectionnée fixée à un instrument d'insertion de cage.

16. Procédé (200) selon la revendication 14 ou 15, comprenant en outre:
la réception d'un entrée utilisateur ajustant au moins l'une parmi la mise en place de cage intersomatique visualisée et la cage intersomatique sélectionnée; et
le recalcul de la mise en place de cage intersomatique et de la forme de la cage en fonction de l'entrée utilisateur; et, éventuellement, dans lequel
l'entrée utilisateur indique également des niveaux de vertèbres spécifiques à fusionner ou un nombre spécifique de niveaux de vertèbres à fusionner.

17. Produit programme informatique, comprenant des instructions qui, lorsqu'elles sont exécutées sur au moins un processeur, amènent l'au moins un processeur à mettre en œuvre le procédé selon l'une quelconque des revendications 1 à 16.

18. Système de planification (100) comprenant au moins un processeur configuré pour mettre en œuvre le procédé (200) selon l'une quelconque des revendications 1 à 16.

19. Système de planification (100) selon la revendication 8, comprenant en outre au moins l'une des entités suivantes:
un dispositif d'imagerie médicale (130) configuré pour générer au moins l'un parmi l'image médicale 2D et l'image 3D du patient;
un dispositif d'entrée utilisateur configuré pour recevoir une entrée utilisateur;
un dispositif d'affichage (120) configuré pour afficher la visualisation.
